# EUROPEAN PATENT SPECIFICATION

(11) **EP 4 606 302 B1**
(45) Date of publication and mention of the grant of the patent: **21.10.2026**
(21) Application number: 25159455.2
(22) Date of filing: 21.02.2025
(51) Int. Cl.: A61B 5/145, A61B 5/00

(54) **METHOD OF DERIVING BIOMETRIC DATA OF ANALYTE MONITORING SYSTEM**
VERFAHREN ZUR ABLEITUNG BIOMETRISCHER DATEN EINES ANALYTÜBERWACHUNGSSYSTEMS
PROCÉDÉ DE DÉRIVATION DE DONNÉES BIOMÉTRIQUES D'UN SYSTÈME DE SURVEILLANCE D'ANALYTE

(30) Priority: 23.02.2024 KR 20240026790
(43) Date of publication of application: 27.08.2025
(60) Divisional application: 26194114.0 / 4 806 340
(73) Proprietor: i-Sens, Inc., Seoul 06646 (KR)
(72) Inventor: LEE, David, Seoul (KR)
(74) Representative: HGF

(56) References cited:
- US-A1- 2019 110 698
- US-A1- 2023 255 518
- US-B2- 11 282 603
- US-B2- 9 320 462

## Description

### BACKGROUND OF THE INVENTION

### 1. Field

The present disclosure relates to a method of deriving biometric data of an analyte monitoring system.

### 2. Description of the Related Art

Recently, with the advancement of medical technology, various medical devices that are attached to a user's body have been developed and sold. A medical device attached to the user's body may be useful for monitoring biometric information or providing treatment by being attached to the skin of a chronic disease patient. For example, chronic diseases such as diabetes require continuous management, and a medical device called glucose meter may be used to monitor glucose in diabetic patients.

There are two types of glucose meters: one that measures blood glucose levels on a one-time basis by drawing blood from the user's fingertip, and one that measures blood glucose levels continuously by attaching the meter to the user's stomach or arm.

As the latter method, a continuous glucose monitoring system (CGMS), which is inserted into the human body and measures glucose levels at intervals of several minutes, has been developed and used. In order to minimize the user's pain and resistance following blood collection, the CGMS may measure glucose continuously after inserting a needle-shaped transdermal sensor into areas where pain is relatively less, such as the stomach, arm, etc.

The CGMS may include a sensor transmitter that is inserted into the user's skin to measure glucose-related biosignals in the body, and a terminal that outputs the received glucose-related biosignals as glucose levels.

The sensor transmitter measures glucose-related biosignals while a part of the sensor is inserted into the human body. Therefore, the human body may recognize the inserted sensor as a foreign substance. This may cause noise to be introduced into the glucose-related biosignals measured by the sensor transmitter. In addition, the sensor may move because the person moves, or the glucose-related biosignals may be affected by surrounding electromagnetic waves when transmitted from the sensor transmitter to the terminal. In such cases, noise may also be introduced into the glucose-related biosignals.

Glucose-related biosignals in which noise is introduced may exhibit a negative intercept phenomenon in which the baseline decreases over time. Conventionally, a preset offset value may be reflected to compensate for this negative intercept phenomenon (decreasing of the baseline). However, the conventional method may have a problem of excessive or insufficient compensation because it uniformly reflects a fixed offset value. In addition, since the offset value must be continuously input from the outside, there may be a problem that the compensation process is inconvenient.

US 2019/110698 A1 relates to apparatuses and methods for processing bio-information of a subject. US 9320462 B2 relates to devices for detecting analyte in body fluid by positioning an analyte sensor in fluid contact with an analyte. US 2023/255518 A1 relates to devices for processing sampled data from analyte sensor, determining a non-attenuated sensitivity value associated with the analyte sensor, determining a representative sensitivity of the sensor for each group of multiple groups of data points from the analyte sensor. US 11282603 B2 relates to a method for minimizing the effect of noise and signal dropouts in a glucose sensor.

### SUMMARY OF THE INVENTION

Against this background, one object of embodiments of the present disclosure is to provide a technology for compensating an analyte-related biosignal in order to accurately output biometric data to a terminal by processing noise introduced into the analyte-related biosignal.

Another object of embodiments of the present disclosure is to provide a technology for compensating the analyte-related biosignal in order to process noise in real time.

The present invention is defined by the independent claim. Further aspects of the present invention are outlined in the dependent claims. According to an embodiment of the present disclosure, there may be provided a method of deriving biometric data of a specific time point displayed on a terminal of an analyte monitoring system, the method including: acquiring a corresponding time point biosignal, which is a biosignal of a time point corresponding to the specific time point; generating a corrected biosignal from the corresponding time point biosignal when the corresponding time point biosignal satisfies a preset condition; determining any one of the corresponding time point biosignal and the corrected biosignal as input data; and deriving biometric data of the specific time point using the input data.

Here, the generating of the corrected biosignal from the corresponding time point biosignal when the preset condition is satisfied may include acquiring an average value for a plurality of biosignals over a predetermined period of time, generating an ideal signal by applying the average value to the corresponding time point biosignal, generating a weight, and generating the corrected biosignal by applying the weight to the corresponding time point biosignal and the ideal signal.

In addition, the generating of the ideal signal may include generating a continuous biosignal with a changing baseline by using the plurality of biosignals including the corresponding time point biosignal, generating a processed continuous biosignal in which a trend is removed from a waveform of the continuous biosignal, and applying the average value to the processed continuous biosignal.

In addition, the generating of the processed continuous biosignal may use a high pass filter (HPF).

In addition, the continuous biosignal may form a constant baseline in a first period and a descending baseline in a second period.

In addition, the generating of the weight may include generating a first weight applied to the corresponding time point biosignal and a second weight applied to the ideal signal, the first weight and the second weight may have a negative correlation, and the generating of the corrected biosignal may include generating the corrected biosignal by adding a first weighted signal obtained by applying the first weight to the corresponding time point biosignal and a second weighted signal obtained by applying the second weight to the ideal signal.

In addition, the first weight and the second weight may have values that vary between 0 (zero) and 1.

In addition, the generating of the corrected biosignal from the corresponding time point biosignal when the corresponding time point biosignal satisfies the preset condition may include determining whether or not the corresponding time point biosignal satisfies the preset condition according to whether or not the corresponding time point of the corresponding time point biosignal has exceeded an end of a preset period of time.

In addition, the determining of any one of the corresponding time point biosignal and the corrected biosignal as the input data may include, when the corresponding time point biosignal satisfies the preset condition and the corrected biosignal is greater than or equal to the corresponding time point biosignal, determining the corrected biosignal as the input data, and when the corresponding time point biosignal does not satisfy the preset condition or the corrected biosignal is smaller than the corresponding time point biosignal, determining the corresponding time point biosignal as the input data.

As described above, according to the embodiments, biometric data may be accurately output to a terminal by processing noise introduced into the analyte-related biosignal.

In addition, according to the embodiments, noise may be processed in real time by reflecting data generated in real time inside the terminal.

In addition, according to the embodiments, since it is not necessary to input a separate reference value from the outside each time to process noise, inconvenience in use may be eliminated.

### BRIEF DESCRIPTION OF THE DRAWINGS

FIG. 1 is a diagram for schematically explaining a continuous glucose monitoring system (CGMS) according to an embodiment.
FIG. 2 is a diagram for explaining an applicator assembly according to an embodiment.
FIG. 3 is a diagram for explaining a process of attaching a sensor transmitter to a human body using an applicator assembly according to an embodiment.
FIG. 4 is a configuration diagram of a sensor transmitter according to an embodiment.
FIG. 5 is a configuration diagram of a terminal according to an embodiment.
FIG. 6 is a flowchart illustrating a method of deriving a glucose value of a CGMS according to an embodiment.
FIG. 7 is a flowchart illustrating a method of generating a corrected biosignal according to an embodiment.
FIG. 8 is an exemplary graph in which blood-related biosignals according to a sensor usage period are continuously connected.
FIG. 9 is an exemplary graph in which a trend is indicated in a continuous biosignal of FIG. 8.
FIG. 10 is an exemplary graph of a continuous biosignal in which a negative intercept phenomenon is indicated.
FIG. 11 is an exemplary graph of a processed continuous biosignal in which a trend is removed and an ideal signal in which an average value is applied to the processed continuous biosignal.
FIG. 12 is a diagram for explaining a process of generating a corrected biosignal of a corresponding time point from a continuous biosignal and an ideal signal.
FIG. 13 is a diagram for explaining weights applied to a continuous biosignal and an ideal signal.

### DETAILED DESCRIPTION

In describing the present disclosure, if it is determined that related known functions may unnecessarily obscure the gist of the present disclosure as they are obvious to those skilled in the art, detailed descriptions thereof will be omitted.

The terms used in the present application are used merely to describe particular embodiments and are not intended to limit the present disclosure. Singular expressions include plural expressions unless the context clearly indicates otherwise. In the present application, it should be understood that terms such as "comprise", "include", or "have" are intended to designate the presence of features, numbers, steps, operations, components, parts, or combinations thereof described in the specification, and they do not preclude the possibility of the presence or addition of one or more other features, numbers, steps, operations, components, parts, or combinations thereof.

In the present specification, an analyte monitoring system according to the present disclosure will be exemplarily described based on the case where it is a continuous glucose monitoring system (CGMS). In other words, the description will be made on the premise that analyte to be monitored by the analyte monitoring system is glucose, and biometric data displayed on a terminal is a glucose value (glucose concentration (including mass concentration and molar concentration)). However, this description does not limit the scope of the analyte monitoring system according to the present disclosure to a continuous glucose monitoring system (CGMS).

Hereinafter, embodiments according to the present disclosure will be described in detail with reference to the accompanying drawings, and in describing with reference to the accompanying drawings, identical or corresponding components will be assigned the same drawing numbers and overlapping descriptions thereof will be omitted.

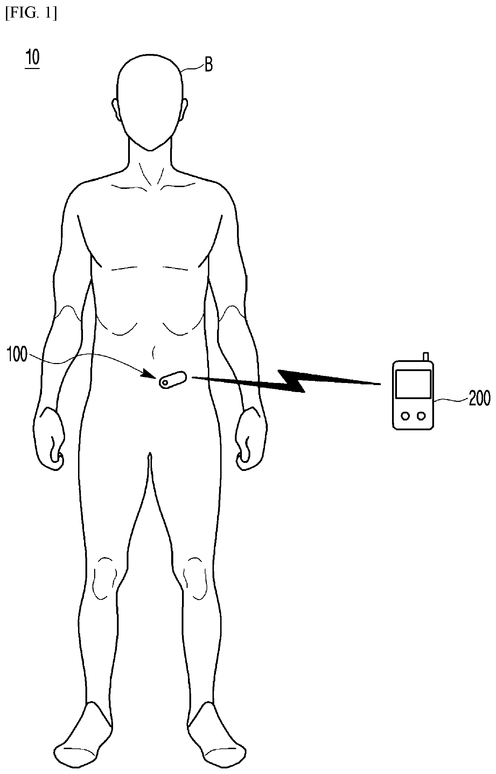

FIG. 1 is a diagram for schematically explaining a CGMS according to an embodiment of the present disclosure.

Referring to FIG. 1, a CGMS 10 (hereinafter referred to as a "system") according to an embodiment may include a sensor transmitter 100 and a terminal 200.

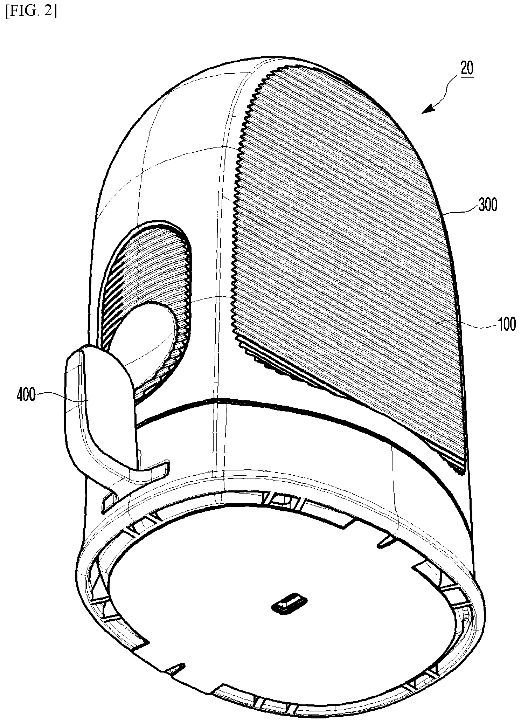

The sensor transmitter 100 may be attached to a human body B by a user manipulating an applicator assembly (20 of FIG. 2), which will be described later. When the sensor transmitter 100 is attached to the human body B, one end of a sensor of the sensor transmitter 100 is inserted into the skin and come into fluid contact with the body fluid of the human body B. The sensor transmitter 100 may generate a glucose-related biosignal from the sensor in fluid contact with the body fluid. For example, one end of the sensor is located in the dermal layer of the skin or the subcutaneous fat layer of the skin and is in fluid connection with a body fluid such as interstitial fluid (ISF), and the sensor transmitter 100 may generate a current value depending on the glucose concentration in the ISF.

The terminal 200 may receive glucose-related biosignals from the sensor transmitter 100 and generate biometric data, such as glucose values, from the glucose-related biosignals and output to the user. The terminal 200 may include, but is not limited to, various devices such as smartphones, mobile phones, tablet PCs, desktops, and laptops, and may have a communication interface capable of communicating with the sensor transmitter 100 and may include a device on which a program or application may be installed.

The sensor transmitter 100 may transmit glucose-related biosignals to the terminal 200 at the request of the terminal 200 or whenever a set data transmission/reception cycle arrives. For data communication between the sensor transmitter 100 and the terminal 200, the sensor transmitter 100 and the terminal 200 may be connected to each other via a wired connection such as a USB cable or wirelessly using methods such as infrared communication, NFC communication, or Bluetooth.

In the present specification, it is assumed that the glucose-related biosignal generated by the sensor transmitter 100 is transmitted to the terminal 200, and it is assumed that biometric data is generated using the glucose-related biosignal received by the terminal 200 and the method of deriving biometric data of the analyte monitoring system according to the present embodiment. However, the scope of the present disclosure is not limited thereto. As another example, the sensor transmitter 100 may generate biometric data using the generated glucose-related biosignal and the method of deriving biometric data of the analyte monitoring system according to the present embodiment, and transmit it to the terminal 200, and the terminal 200 may display the biometric data received from the sensor transmitter 100 to the user and/or process the biometric data received from the sensor transmitter 100 and display the processed biometric data to the user.

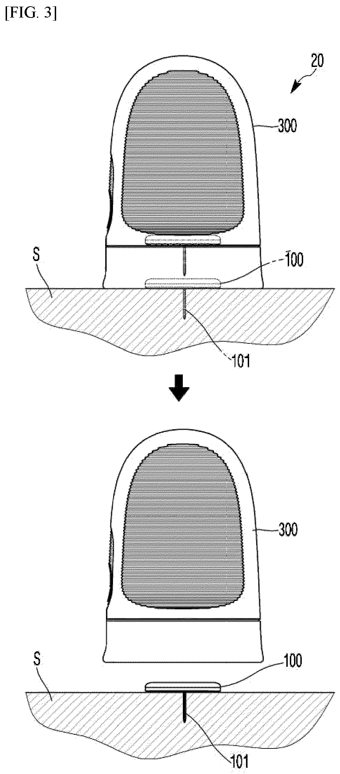

FIG. 2 is a diagram for explaining an applicator assembly according to an embodiment. FIG. 3 is a diagram for explaining a process of attaching a sensor transmitter to a human body using an applicator assembly according to an embodiment.

Referring to FIG. 2 and FIG. 3, an applicator assembly 20 according to an embodiment includes the sensor transmitter 100, an applicator 300, and a protective member 400.

The sensor transmitter 100 is initially (for example, it may mean the time of purchase of the applicator assembly 20 by the user) fixed inside the applicator 300 and may be discharged to the outside of the applicator 300 when the user manipulates the applicator 300.

The applicator 300 operates to discharge the sensor transmitter 100 inside to the outside and attach it to a specific body part of the user through manipulation by the user. The applicator 300 is formed in a shape with one side open, and the sensor transmitter 100 is discharged to the outside of the applicator 300 through the open side of the applicator 300.

For example, the applicator 300 may include a needle (not shown) formed to surround one end of a sensor 101 of the sensor transmitter 100 to insert the sensor 101 into the skin, a first elastic member (not shown) that pushes the needle (not shown) and the sensor transmitter 100 together toward skin S, and a second elastic member (not shown) for pulling out only the needle (not shown) from the skin S. When the user manipulates the applicator assembly 20, the first elastic member (not shown) inside the applicator 300 is elastically deformed so that the needle (not shown) and one end of the sensor 101 may be inserted into the skin. When one end of the sensor 101 is inserted into the skin, only the needle (not shown) may be pulled out due to the elastic deformation of the second elastic member (not shown). In this case, the user may safely and easily attach the sensor transmitter 100 to the skin S through the applicator 300 and insert one end of the sensor 101 into the human body B. For example, the first elastic member (not shown) in its initial compressed state may be released from the compressed state by the user's manipulation of the applicator 300, thereby pushing the needle (not shown) and the sensor transmitter 100 together toward the skin S. For example, the second elastic member (not shown) in its initial compressed state may be released from the compressed state, thereby allowing only the needle (not shown) to be pulled out from the human body B. For example, the second elastic member (not shown) in its initial tensioned state may be released from the tensioned state, thereby allowing only the needle (not shown) to be removed from the human body B.

As another example, the applicator may include a first part and a second part that are relatively movable, and may not include the first elastic member described above. In this case, when the user moves the first part relative to the second part, the sensor transmitter coupled to the first part may be attached to the skin, and the needle and one end part of the sensor may be inserted into the human body. Here, when it is said that the user relatively moves the first part with respect to the second part, it may mean, for example, that the user presses the first part in the upper-to-lower direction, so that the displacement between the upper end part of the first part and the upper end part of the second part decreases.

The protective member 400 prevents the sensor transmitter 100 from moving from the first position inside the applicator 300 to the second position of the applicator 300. The first position of the sensor transmitter 100 is located on the upper side of the applicator 300 compared to the second position of the sensor transmitter 100. The first position may refer to the position of the sensor transmitter 100 before the user operates the applicator 300 to trigger the sensor transmitter 100. The second position may refer to the position of the sensor transmitter 100 after the user operates the applicator 300 to trigger the sensor transmitter 100. The protective member 400 prevents the sensor transmitter 100 from being triggered due to user carelessness or the like. The user may attach the sensor transmitter 100 to the skin S only after removing the protective member 400 coupled to the applicator 300.

The user's manipulation process of the applicator assembly 20 to attach the sensor transmitter 100 to the human body B will be described in more detail. The user removes the protective member 400 from the applicator assembly 20 and closely adheres the open surface of the applicator 300 to a specific area of the skin S on the human body B. When the user manipulates the applicator 300 while keeping it closely adhered to the skin S of the human body B, the sensor transmitter 100 disposed inside the applicator 300 may be discharged through the open surface of the applicator 300 and attached to the skin S. Since one end of the sensor 101 is disposed in a form exposed from the lower surface of the sensor transmitter 100 at the lower part of the sensor transmitter 100, one end of the sensor 101 may be inserted into the skin S together with the needle (not shown) provided in the applicator 300. In other words, the sensor transmitter 100 may be attached to the skin S with one end of the sensor 101 inserted into the skin S. Thereafter, when the second elastic member (not shown) is elastically deformed, only the needle (not shown) may be pulled out from the skin S.

An adhesive tape nay be provided on the surface of the sensor transmitter 100 that contacts the human body B so that the sensor transmitter 100 may be fixedly attached to the skin S of the human body B. Accordingly, when the user spaces apart the applicator 300 from the skin S of the human body B after the above-described process, the sensor transmitter 100 is fixedly attached to the skin S of the human body B by the adhesive tape, and the applicator 300 may be removed from the skin S.

Although FIG. 3 illustrates that the sensor transmitter 100 is a single member that moves in the downward direction, the scope of the present disclosure is not limited thereto. As another non-limiting example, the sensor transmitter may include at least two sub-components spaced apart from each other within the applicator 300, and may be attached to the skin S in a form in which the at least two sub-components are coupled to form one sensor transmitter by the user's manipulation of the applicator 300. In other words, the sensor transmitter may include a first sub-component including a sensor, and a second sub-component including the remaining components of the sensor transmitter excluding the sensor, and the first and second sub-components may be initially spaced apart from each other within the applicator 300, but the first sub-component may be moved toward the second sub-component by a user's manipulation of the applicator 300 so that the first and second sub-components may be coupled to each other. In this example, the first elastic member described above may move the first sub-component and the needle in the downward direction.

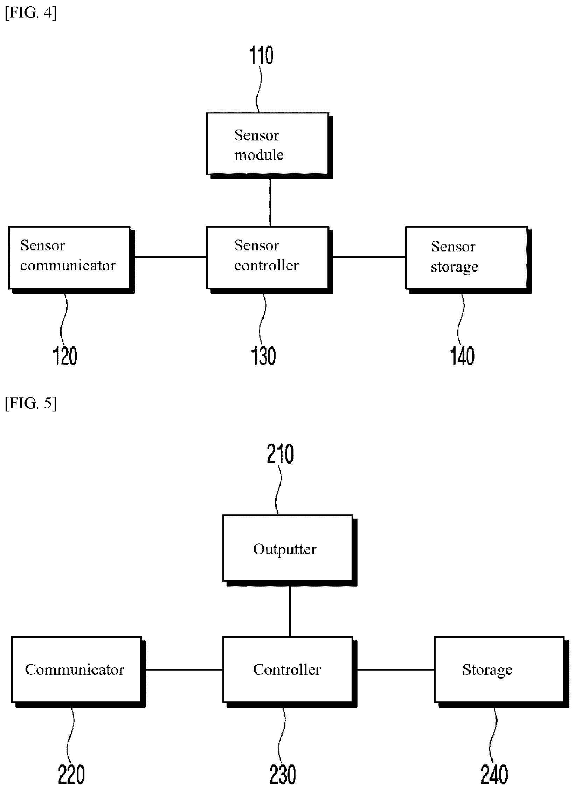

FIG. 4 is a configuration diagram of a sensor transmitter according to an embodiment.

Referring to FIG. 4, the sensor transmitter 100 according to an embodiment may include a sensor module 110, a sensor communicator 120, a sensor controller 130, and a sensor storage 140.

The sensor module 110 may include at least one sensor that is inserted into the skin of a human body to sense a biomass. The at least one sensor may be inserted into the skin of a human body to sense a biomass for, for example, 7 days, 14 days, 15 days, or 20 days. The biomass may be a potential difference depending on the glucose concentration in the ISF. The sensor module 110 may generate a biosignal for the biomass measured by the at least one sensor. When the biomass measured by the sensor is an analog signal that is a potential difference depending on the glucose concentration in the ISF, the sensor module 110 may generate a glucose-related biosignal. The glucose-related biosignal may be a current value. As a non-limiting example, the sensor module 110 may generate one 10-second unit glucose-related biosignal every 10 seconds. The 10-second unit glucose-related biosignal may be generated by, for example, generating a plurality of glucose-related biosignals for 10 seconds and then calculating the arithmetic mean of the middle range glucose-related biosignals excluding the upper 30% of glucose-related biosignals and the lower 30% of glucose-related biosignals among the plurality of glucose-related biosignals. The plurality of glucose-related biosignals generated for 10 seconds may be, for example, 30 or 90.

The sensor communicator 120 may exchange data or information with the terminal (200 of FIG. 1). For example, the sensor communicator 120 may transmit a glucose-related biosignal received from the sensor module 110 or a glucose-related biosignal stored in the sensor storage 140 to the terminal (200 of FIG. 1). The sensor communicator 120 may exchange data or information with the terminal (200 of FIG. 1) at substantially regular time intervals (data transmission/reception cycle). For example, the sensor communicator 120 may transmit six 10-second unit glucose-related biosignals belonging to a 1-minute data collection section collected and stored in the sensor storage 140 in the form of one data packet to the terminal (200 of FIG. 1) at substantially 1-minute intervals of data transmission/reception cycles. Alternatively, the sensor communicator 120 may transmit thirty 10-second unit glucose-related biosignals belonging to a 5-minute data collection section collected and stored in the sensor storage 140 in the form of one data packet to the terminal (200 of FIG. 1) at substantially 5-minute intervals of data transmission/reception cycles.

If the sensor communicator 120 failed to transmit glucose-related biosignals belonging to the previous data collection section that should have been transmitted when the previous data transmission/reception cycle arrived to the terminal (200 of FIG. 1) (this also includes a case where the sensor communicator transmitted a data packet composed of glucose-related biosignals belonging to the previous data collection section to the terminal when the previous data transmission/reception cycle arrived, but the terminal failed to receive the data packet), the sensor communicator 120 may transmit glucose-related biosignals belonging to the previous data collection section when the next data transmission/reception cycle arrives, or transmit glucose-related biosignals belonging to the previous data collection section when the next data transmission/reception sub-cycle arrives. In the former case, the sensor communicator 120 may transmit at least two data packets together to the terminal (200 of FIG. 1) when the next data transmission/reception cycle arrives. At least two data packets include a data packet including glucose-related biosignals belonging to the previous data collection section that should have been transmitted in the previous data transmission/reception cycle, and a data packet including glucose-related biosignals belonging to the next data collection section that is scheduled to be transmitted at the arrival time of the next data transmission/reception cycle. In the latter case, the sensor communicator 120 may transmit only data packets for glucose-related biosignals belonging to the previous data collection section when the next data transmission/reception sub-cycle arrives. Here, the data transmission/reception sub-cycle may refer, for example, to a cycle in which the sensor communicator 120 performs ADV to check the connection status with the terminal when the sensor communicator 120 and the communicator (220 of FIG. 5) of the terminal are connected via Bluetooth. As a non-limiting example, the ADV performance cycle of the sensor communicator 120 may arrive every minute.

Hereinafter, for the convenience of explanation, it is assumed that 'a plurality of 10-second unit glucose-related biosignals (thirty 10-second unit glucose-related biosignals) belonging to a 5-minute data collection section' are stored in the sensor storage 140. In addition, hereinafter, it is assumed that the 'plurality of 10-second unit glucose-related biosignals belonging to the 5-minute data collection section' are transmitted from the sensor communicator 120 to the terminal (200 of FIG. 1) in the form of one data packet every time a data transmission/reception cycle of 5 minutes arrives. In addition, hereinafter, the description assumes that the glucose value derived using the one data packet is displayed on the terminal (200 of FIG. 1) as the value of a specific time point, which is a representative time point of the data collection section, which is the 5-minute section. More specifically, it is assumed that thirty 10-second unit glucose-related biosignals belonging to a 5-minute data collection section from 16:05 on September 10, 2023 to 16:10 on September 10, 2023 are transmitted to the terminal (200 in FIG. 1) in the form of one data packet at 16:10 on September 10, 2023, which is the arrival time point of a 5-minute data transmission/reception cycle, and that the glucose value derived using the one data packet is displayed on the terminal (200 in FIG. 1) as that at 16:10 on September 10, 2023, which is a representative time point of the 5-minute data collection section. However, such description does not limit the scope of the present disclosure.

The sensor controller 130 may control the overall configuration of the sensor transmitter 100 including the sensor module 110, the sensor storage 140, and the sensor communicator 120. For example, the sensor controller 130 may receive a control signal from the terminal (200 of FIG. 1) and control the configuration of the sensor transmitter 100 accordingly.

The sensor storage 140 may store data or information. For example, the sensor storage 140 may store a 10-second unit glucose-related biosignal generated by the sensor module 110. As a non-limiting example, the 10-second unit glucose-related biosignal stored in the sensor storage 140 may include 10-second unit glucose-related biosignals belonging to a data collection section including the current time point. Specifically, if the last time point the sensor communicator 120 transmitted thirty 10-second unit glucose-related biosignals to the terminal (200 in FIG. 1) in the form of one data packet was 16:05 on September 10, 2023, the sensor storage 140 may store a total of thirty 10-second unit glucose-related biosignals from 16:05 on September 10, 2023 to 16:10 on September 10, 2023. The thirty 10-second unit glucose-related biosignals stored in the sensor storage 140 from 16:05 on September 10, 2023 to 16:10 on September 10, 2023 may be selectively deleted from the sensor storage 140 after being transmitted to the terminal (200 of FIG. 1) at 16:10 on September 10, 2023. In the sensor storage 140, data (e.g., a command value of a control signal) received from the terminal (200 of FIG. 1) may be stored.

FIG. 5 is a configuration diagram of a terminal according to an embodiment.

Referring to FIG. 5, the terminal (200 of FIG. 1) according to an embodiment may include an outputter 210, a communicator 220, a controller 230, and a storage 240.

The outputter 210 may output biometric data (e.g., glucose information) derived by the controller 230 so that a user may check it. Specifically, the outputter 210 may display glucose information as a numerical value (value) based on a specific time point or, further, a graph processed from the numerical value. For example, the outputter 210 may display biometric data as a glucose value, and may display the glucose value while changing the reference time point at substantially regular intervals.

The communicator 220 may communicate with the sensor communicator (120 of FIG. 4) of the sensor transmitter and exchange data or information. For example, the communicator 220 may receive at least one data packet including a plurality of 10-second unit glucose-related biosignals from the sensor communicator (120 of FIG. 4) every time a data transmission/reception cycle of 5 minutes arrives. In addition, the communicator 220 may transmit a control signal for controlling the sensor transmitter (100 of FIG. 1) to the terminal (200 of FIG. 1).

Data or information may be stored in the storage 240. For example, a plurality of 10-second unit glucose-related biosignals transmitted from the sensor transmitter (100 of FIG. 1) to the terminal (200 of FIG. 1) may be stored in the storage 240. In addition, the storage 240 may store a plurality of preprocessed 10-second unit glucose-related biosignals that have undergone preprocessing (primary noise removal) by the controller 230. In addition, the storage 240 may store at least one 1-minute unit glucose-related biosignal generated by the controller 230. In addition, the storage 240 may store at least one 5-minute unit glucose-related biosignal generated by the controller 230. In addition, the storage 240 may store at least one corrected biosignal, which is a correction of at least one 5-minute unit glucose-related biosignal. In addition, the storage 240 may store data input by the user or environment setting data for setting the operating environment of the terminal (200 of FIG. 1). The 1-minute unit glucose-related biosignal, the 5-minute unit glucose-related biosignal, and the corrected biosignal will be described later.

The controller 230 may derive a glucose value displayed on the terminal as corresponding to a specific time point by using a plurality of 10-second unit glucose-related biosignals belonging to a specific data collection section received from the sensor transmitter (100 of FIG. 1). For example, the controller 230 may derive a glucose value displayed on the terminal as corresponding to 16:10 on September 10, 2023 by using thirty 10-second unit glucose-related biosignals belonging to a data collection section from 16:05 on September 10, 2023 to 16:10 on September 10, 2023.

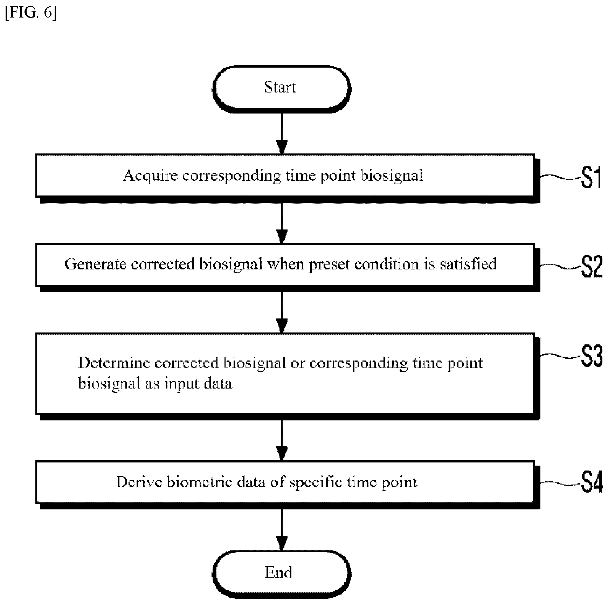

FIG. 6 is a flowchart illustrating a method of deriving a glucose value of a CGMS according to an embodiment.

Referring to FIG. 6, the controller 230 may acquire a corresponding time point biosignal (S1). To this end, the controller 230 may perform the following series of processes.

First, the controller 230 may preprocess (primary noise removal) thirty 10-second unit glucose-related biosignals to generate thirty preprocessed 10-second unit glucose-related biosignals. To this end, the controller 230 may use a known signal preprocessing technique, such as a Savitzky-Golay filter or the like.

Next, the controller 230 may generate five 1-minute unit glucose-related biosignals from the thirty preprocessed 10-second unit glucose-related biosignals. For example, a 1-minute unit glucose-related biosignal may be derived by grouping preprocessed 10-second unit glucose-related biosignals into 1-minute intervals (one 1-minute unit group is composed of six preprocessed 10-second unit glucose-related biosignals), and taking the arithmetic mean of the four preprocessed 10-second unit glucose-related biosignals, excluding one upper value and one lower value among the six preprocessed 10-second unit glucose-related biosignals within the 1-minute unit group.

In other words, in the above example, the controller 230 may generate a total of five 1-minute unit glucose-related biosignals, which are composed of one 1-minute unit glucose-related biosignal for 1 minute from 16:05 to 16:06 on September 10, 2023 (the corresponding time point of the 1-minute unit glucose-related biosignal is 16:05:30 on September 10, 2023, which is the middle time point of the section), one 1-minute unit glucose-related biosignal for 1 minute from 6 minutes to 7 minutes (the corresponding time point of the 1-minute unit glucose-related biosignal is 16:06:30 on September 10, 2023, which is the middle time point of the section), one 1-minute unit glucose-related biosignal for 1 minute from 7 minutes to 8 minutes (the corresponding time point of the 1-minute unit glucose-related biosignal is 16:07:30 on September 10, 2023, which is the middle time point of the section), and one 1-minute unit glucose-related biosignal for 1 minute from 8 minutes to 9 minutes (the corresponding time point of the 1-minute blood sugar-related biosignal is 16:08:30 on September 10, 2023, which is the middle point of the section), and one 1-minute unit glucose-related biosignal for 1 minute from 09 minutes to 10 minutes (the corresponding time point of the 1-minute unit glucose-related biosignal is 16:09:30 on September 10, 2023, which is the middle time point of the section).

In addition, the controller 230 may generate a 5-minute unit glucose-related biosignal from the total of five 1-minute unit glucose-related biosignals. For example, a 5-minute unit glucose-related biosignal may be derived by taking the arithmetic mean of the three 1-minute unit glucose-related biosignals excluding one upper value and one lower value of the five 1-minute unit glucose-related biosignals. In other words, in the above example, the controller 230 may generate one 5-minute unit glucose-related biosignal for 5 minutes from 16:05 to 16:10 on September 10, 2023. The corresponding time point of the 5-minute unit glucose-related biosignal may be 16:07:30 on September 10, 2023, which is the middle time point of the section.

In the above example, the controller 230 may derive the glucose value displayed on the terminal as that of 16:10 on September 10, 2023 based on the 5-minute unit glucose-related biosignal having a corresponding time point of 16:07:30 on September 10, 2023. In this case, the controller 230 may acquire the 5-minute unit glucose-related biosignal having a corresponding time of 16:07:30 on September 10, 2023 as the corresponding time point biosignal of step S1.

Next, the controller 230 may generate a corrected biosignal from the corresponding time point biosignal when the corresponding time point biosignal satisfies a preset condition (S2). In other words, the controller 230 may generate the corrected biosignal by correcting the corresponding time point biosignal. For example, the controller 230 may generate a corrected biosignal from the 5-minute unit glucose-related biosignal if the 5-minute unit glucose-related biosignal satisfies a preset condition. For example, the controller 230 may not generate a corrected biosignal from the 5-minute unit glucose-related biosignal if the 5-minute unit glucose-related biosignal does not satisfy the preset condition. At this time, 'the 5-minute unit glucose-related biosignal satisfies the preset condition' may mean that the corresponding time point of the 5-minute unit glucose-related biosignal has exceeded the end of the preset period of time. Here, the preset period of time may be '5 days from the sensor usage time point' or '9 days from the sensor usage time point.' The 'sensor usage time point' may mean the time point of a 10-second section for generating the first 10-second unit glucose-related biosignal, or may mean the time point when power is supplied to the sensor transmitter.

For example, if the sensor starts sensing glucose-related biovalues from 00:00 on September 1, 2023, and the preset period of time is '9 days from the sensor usage start time point', the corresponding time point biosignal acquired in step S1 (the 5-minute unit glucose-related biosignal whose corresponding time point is 16:07:30 on September 10, 2023) satisfies the preset condition. Therefore, the controller 230 may generate a corrected biosignal based on the 5-minute unit glucose-related biosignal whose corresponding time point is 16:07:30 on September 10, 2023. The method of generating the corrected biosignal will be described later.

Next, the controller 230 may determine any one of the corresponding time point biosignal and the corrected biosignal as input data (S3).

If the corresponding time point biosignal does not satisfy the preset condition and the controller 230 does not generate the corrected biosignal for the corresponding time point biosignal, the controller 230 may determine the corresponding time point biosignal as input data. Based on the above-described example, the 5-minute unit glucose-related biosignal whose corresponding time point is 16:07:30 on September 5, 2023 does not satisfy the preset condition, and therefore the controller 230 may not generate a corrected biosignal for the 5-minute unit glucose-related biosignal whose corresponding time point is 16:07:30 on September 5, 2023. In this case, the controller 230 may determine the 5-minute unit glucose-related biosignal whose corresponding time point is 16:07:30 on September 5, 2023 as input data.

If the corresponding time point biosignal satisfies the preset condition and the controller 230 generates a corrected biosignal for the corresponding time point biosignal, the controller 230 may compare the size of the corresponding time point biosignal (current value) and the corrected biosignal (current value) to determine a signal having a relatively large value as input data. Based on the above-described example, since the 5-minute unit glucose-related biosignal whose corresponding time point is 16:07:30 on September 10, 2023 satisfies the preset condition, the controller 230 may generate a corrected biosignal for the 5-minute unit glucose-related biosignal whose corresponding time point is 16:07:30 on September 10, 2023. If the value of the generated corrected biosignal (for example, 6 nA) is greater than or equal to the value of the 5-minute unit glucose-related biosignal (for example, 2.5 nA) whose corresponding time point is 16:07:30 on September 10, 2023, the controller 230 may determine the corrected biosignal as input data. If the value of the generated corrected biosignal (for example, 2 nA) is less than the value of the 5-minute unit glucose-related biosignal (for example, 2.5 nA) whose corresponding time point is 16:07:30 on September 10, 2023, the controller 230 may determine the 5-minute unit glucose-related biosignal whose corresponding time point is 16:07:30 on September 10, 2023, as input data.

Next, the controller 230 may derive biometric data of a specific time point corresponding to the corresponding time point of the corresponding time point biosignal using the input data (S3).

In this step, biometric data may be derived using the input data and a preset formula. In the above-described example where the corresponding time point is 16:07:30 on September 10, 2023 and the size of the corresponding time point biosignal is greater than the size of the corrected biosignal, the input data may be determined as 6nA, which is the value of the corrected biosignal. The preset formula may be a relationship between the current value, which is the input data of the corresponding time point, and the first biometric data (glucose value) of the specific time point. In this case, using the input data (6 nA) determined for the corresponding time point of 16:07:30 on September 10, 2023 and the preset formula, the first biometric data (glucose value, for example, 125 mg/dL) whose specific time point is 16:10 on September 10, 2023 may be derived. As another example, the preset formula may be a relationship between the current value, which is the input data of the corresponding time point, and the second biometric data (glucose concentration in ISF) of the corresponding time point. In this case, using the input data (6nA) determined for the corresponding time point of 16:07:30 on September 10, 2023 and the preset formula, the second biometric data (glucose concentration in ISF, for example, 120 mg/dL) whose corresponding time point is 16:07:30 on September 10, 2023 may be derived. If the result value of the preset formula is other biometric data rather than a glucose value, the controller 230 may derive the glucose value of a specific time point from the other biometric data of the corresponding time point that has been calculated. For example, if the other biometric data that has been calculated is the glucose concentration in ISF, the controller 230 may derive the glucose value of a specific time point from the glucose concentration in the ISF of the corresponding time point that has been calculated using a known technology (e.g., a Kalman filter) that considers the concentration and concentration gradient of glucose in the ISF and glucose in the blood vessels, respectively. The glucose value derived through the above process may be displayed on the terminal as a value of a specific time point.

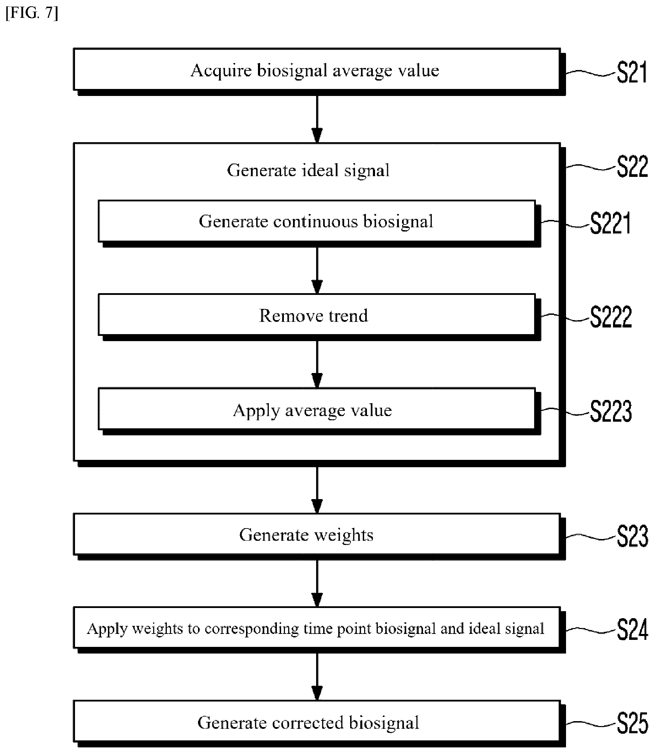

FIG. 7 is a flowchart illustrating a method of generating a corrected biosignal according to an embodiment.

Referring to FIGS. 6 and 7 and the contents described above, the controller 230 may correct the corresponding time point biosignal if the corresponding time point of the corresponding time point biosignal exceeds the end of the preset period of time. Specifically, the controller 230 corrects the corresponding time point biosignal under a specific condition to compensate for the negative intercept phenomenon of blood-related biosignals according to the passage of sensor usage time.

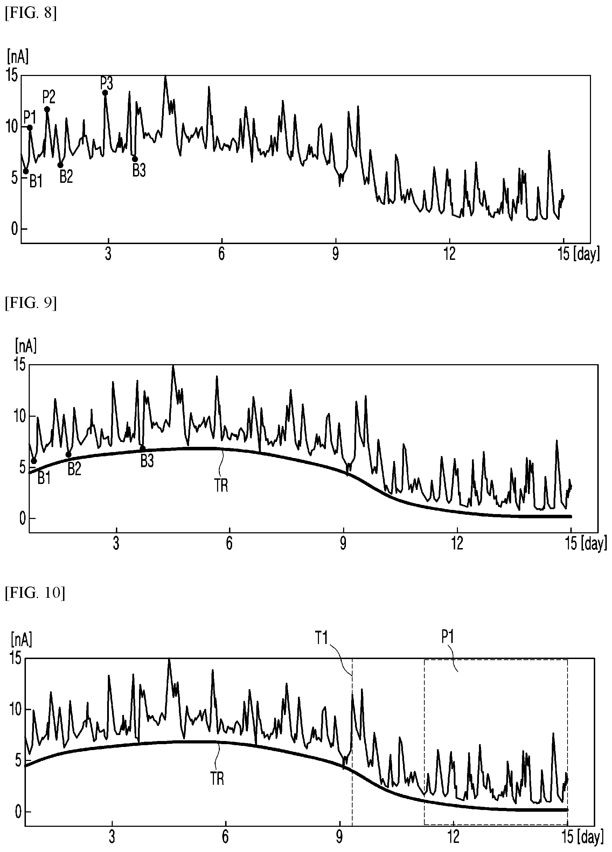

Hereinafter, the negative intercept phenomenon of the blood-related biosignals will be described with reference to FIGS. 8 to 10.

FIG. 8 is an exemplary graph in which blood-related biosignals according to a sensor usage period are continuously connected. Specifically, FIG. 8 continuously shows a plurality of 5-minute unit blood-related biosignals acquired every 5 minutes over the entire sensor usage period (15 days) by connecting them (continuous biosignal). In FIG. 8, the horizontal axis (X-axis) represents the sensor usage period (day).

Referring to FIG. 8, the 5-minute unit blood-related biosignals may be expressed as current values over the entire sensor usage period (15 days). For example, if the glucose value of a first specific time point is high, the 5-minute unit blood-related biosignal of the first corresponding time point corresponding to the first specific time point has a high current value and forms a peak in the continuous biosignal. For example, if the glucose value of a second specific time point is low, the 5-minute unit blood-related biosignal of the second corresponding time point corresponding to the second specific time point has a low current value and forms a bottom in the continuous biosignal. The continuous biosignal in which the 5-minute unit blood-related biosignals are connected may show a peak or bottom at a specific time point depending on the glucose value for several days. In this drawing, P1, P2, and P3 may represent peak values, and B1, B2, and B3 may represent bottom values, respectively, and a greater number of peak values and bottom values may appear as a period of time passes.

In FIG. 8, the continuous biosignal has a waveform with a current value starting from about 5 nA and up to 15 nA, and may have a waveform with a bottom value below 5 nA after about 9 days of sensor usage period. Then, after about 12 days of sensor usage period, the bottom value drops to around 0 nA, and may maintain the lowered bottom value thereafter.

FIG. 9 is an exemplary graph in which a trend is indicated in a continuous biosignal of FIG. 8. In FIG. 9, the horizontal axis (X-axis) represents the sensor usage period (day).

Referring to FIG. 9, as described above, the continuous biosignal may have a bottom value that is somewhat constant although there are fluctuations until the sensor usage period exceeds 9 days. However, after the sensor usage period exceeds 9 days, the bottom value shows a decreasing pattern, and after the sensor usage period exceeds 12 days, the decreased bottom value may be maintained constant. As such, when the bottom value of the continuous biosignal changes by decreasing or increasing, a trend may be formed. In this drawing, the trend may be represented as a line in which the bottom values in the waveform of the continuous biosignal are connected and may be defined as TR. Usually, even if the glucose value decreases, the current value of the 5-minute unit glucose-related biosignal of the time point corresponding thereto does not fall below the trend, so a kind of baseline for the current value may be formed around the trend.

FIG. 10 is an exemplary graph of a continuous biosignal in which a negative intercept phenomenon is indicated. In FIG. 10, the horizontal axis (X-axis) represents the sensor usage period (day).

Referring to FIG. 10, the negative intercept phenomenon may be understood as the bottom value in the continuous biosignal decreasing as the sensor usage period increases. As described above, the fluctuating bottom value generates a trend of the continuous biosignal, and the negative intercept phenomenon may mean that the progress of this trend is gradually moving downwards. Then, the baseline also gradually moves downwards. If the sensor usage period increases further, the baseline in a lowered state may remain relatively constant despite the passage of time. The maintenance of the phenomenon where the baseline decreases is named negative intercept stabilization, and the section may be named as the negative intercept stabilization section.

For example, the continuous biosignal may begin to show a decrease in the baseline starting from time point T1, when the sensor usage period exceeds 9 days (the onset of the negative intercept phenomenon). Then, when the sensor usage period exceeds 12 days, the decreasing baseline may remain as it is (the negative intercept stabilization is reached). The continuous biosignal may exhibit the negative intercept stabilization section during the P1 section.

If the negative intercept phenomenon occurs like this, the terminal may output an inaccurate glucose value. This is because the current value decreases significantly as the negative intercept increases, and hypoglycemia cannot be properly expressed. The terminal may show good sensitivity by showing peaks and bottoms. However, hypoglycemia may be calculated as a lower glucose value and output to the user, or the terminal may generate an error by calculating data based on the inaccurate current value.

Conventionally, negative intercepts could be removed only by using a fixed offset value or by acquiring an offset value from the outside. However, in the present disclosure, the terminal may solve this conventional problem by applying a weight that changes over time to the corresponding time point biosignal and ideal signal without external input, thereby generating a corrected biosignal. Hereinafter, a series of processes for generating a corrected biosignal will be described.

Referring again to FIG. 7, first, the controller 230 may acquire an average value for a plurality of glucose-related biosignals over a predetermined period of time (S21). Here, the predetermined period of time may mean a section before the negative intercept phenomenon described above occurs. In other words, based on the example described above, the plurality of glucose-related biosignals used to acquire the average value may be 5-minute unit glucose-related biosignals before the sensor usage period exceeds 9 days. For example, the average value may be an arithmetic mean of the current values of all 5-minute unit glucose-related biosignals before the sensor usage period exceeds 9 days. As another example, the average value may be an arithmetic mean of the remaining values excluding the upper 30% and lower 30% of the current values of all 5-minute unit glucose-related biosignals before the sensor usage period exceeds 9 days.

Next, the controller 230 may generate an ideal signal (S22).

In order to generate the ideal signal, the controller 230 may connect all of the plurality of 5-minute unit biosignals from the sensor usage start time point to the corresponding time point to generate the continuous biosignal with a changing baseline (S221), generate a processed continuous biosignal by removing a trend from the continuous biosignal (S222), and generate the ideal signal by applying the average value to the processed continuous biosignal (S223).

The controller 230 may generate the processed continuous biosignal by removing the trend from the continuous biosignal using a filter. For example, the controller 230 may generate a processed continuous biosignal, which is an output value from which a trend has been removed, by inputting a continuous biosignal to a filter including at least one of a low pass filter (LPF), a high pass filter (HPF), a band pass filter (HPF), a band rejection filter (BRF), a fiite impulse response (FIR) filter, and an infinite impulse response (IIR) filter. As a non-limiting example, the controller may remove the trend of the continuous biosignal using the HPF. The cutoff frequency of the HPF may be, for example, 0.3 µHz (period: 38.6 days), but the scope of the present disclosure is not limited thereto.

The controller 230 may generate weights and apply the weights to the continuous biosignal and the ideal signal, respectively. The controller 230 may generate a corrected continuous biosignal as a result of the application of the weights. When the terminal detects a negative intercept phenomenon, the terminal may apply the first and second weights having a negative correlation to the continuous biosignal and the ideal signal and combine the results to generate a corrected continuous biosignal. When the intercept decreases to a negative value over time, the terminal may also change the first and second weights over time.

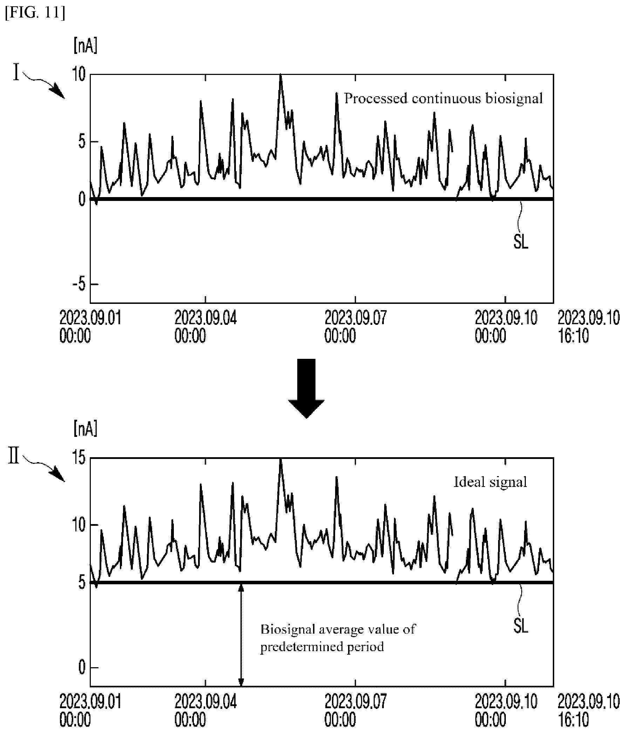

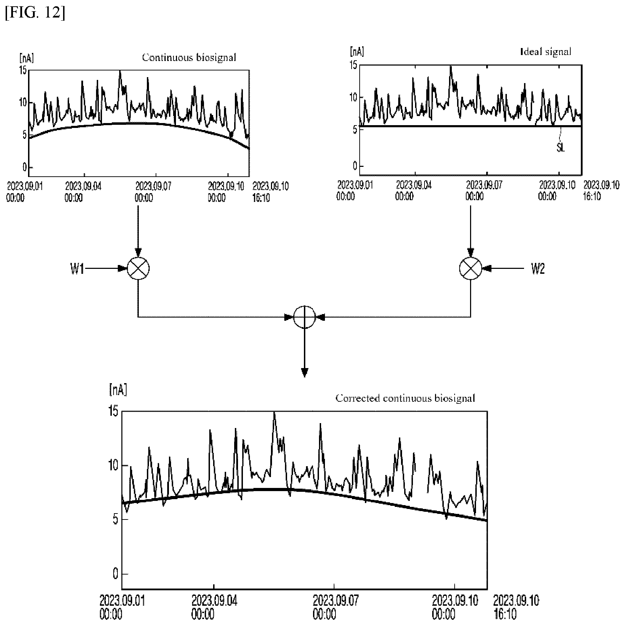

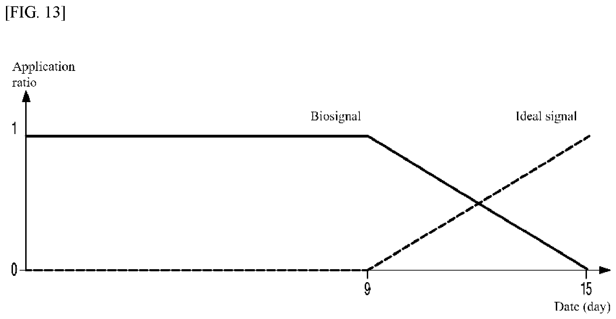

FIG. 11 is an exemplary graph of a processed continuous biosignal in which a trend is removed and an ideal signal in which an average value is applied to the processed continuous biosignal. FIG. 12 is a diagram for explaining a process of generating a corrected biosignal of a corresponding time point from a continuous biosignal and an ideal signal. FIG. 13 is a diagram for explaining weights applied to a continuous biosignal and an ideal signal. In FIGS. 11 and 12, the horizontal axis (X-axis) represents the time from 00:00 on September 1, 2023 to the present time point (the arrival time point of the most recent data transmission/reception cycle between the sensor transmitter and the terminal).

The controller 230 may generate a continuous biosignal using the 5-minute unit biosignal of the time point closest to the present time point and the 5-minute unit biosignals received from the previously stored sensor usage time point (step S221 of FIG. 7). Such a continuous biosignal may include a trend as described above.

Next, the controller 230 may generate a processed continuous biosignal by removing a trend from the continuous biosignal (step S222 of FIG. 7) and may generate an ideal signal by applying an average value to the processed continuous biosignal (step S223 of FIG. 7).

Referring to FIG. 11, the ideal signal and the formation process thereof may be illustrated. The controller 230 may generate a processed continuous biosignal having waveform I by removing a trend from the continuous biosignal. The processed continuous biosignal may have generally similar bottom values. These bottom values may similarly converge to a single straight line, which is defined as a baseline hereinafter and may be illustrated as SL in this drawing. The baseline (SL) may have a single current value. Since the processed continuous biosignal has a trend removed from the continuous biosignal, the reference value may be formed as 0 (zero) first.

Next, the controller 230 may apply the average value to the processed continuous biosignal to finally generate the ideal signal. When the average value is applied to the processed continuous biosignal, the baseline (SL) may rise. In other words, the average value may raise the baseline. The controller 230 may adjust the processed continuous biosignal so that the waveform of the ideal signal (or its waveform) reaches the height formed by the continuous biosignal for the first 9 days. Then, the controller 230 may finally generate the ideal signal having waveform II.

Returning to FIG. 7, the controller 230 may generate weights (S23) and apply the weights to the continuous biosignal and the ideal signal, respectively (S24). Therefore, the controller 230 may generate a corrected continuous biosignal as a result of the weight application (S25). The controller 230 may generate a plurality of weights, and may generate a first weight applied to the continuous biosignal and a second weight applied to the ideal signal. The controller 230 may apply the first weight to the continuous biosignal and the second weight to the ideal signal to generate a corrected continuous biosignal, which is a continuous biosignal from which the negative intercept phenomenon has been removed. The controller 230 may obtain a corrected biosignal (current value, Y-axis value of the rightmost point on the X-axis of the corrected continuous biosignal of FIG. 12) in which a 5-minute unit glucose-related biosignal of a corresponding time point is corrected from the corrected continuous biosignal.

In order to generate the corrected continuous biosignal, the controller 230 may apply the first and second weights that change over time to the continuous biosignal and the ideal signal, and generate the corrected continuous biosignal by combining the results. Specifically, referring to FIG. 12, the controller 230 may apply the first weight (W1) to the continuous biosignal and the second weight (W2) to the ideal signal, respectively. The controller 230 may add the result of applying the first weight (W1) to the continuous biosignal and the result of applying the second weight (W2) to the ideal signal. As a result, the controller 230 may finally generate a corrected continuous biosignal. In the corrected continuous biosignal, the change (curvature) of the baseline may be formed more gradually than that in the continuous biosignal, because the ideal signal is reflected. As the value to which the weight is applied to the ideal signal is reflected, the baseline of the compensated corrected continuous biosignal in the negative intercept occurrence section and/or the negative intercept stabilization section gradually increases and may be more gently bent than the baseline of the continuous biosignal.

In particular, the first weight (W1) and the second weight (W2) applied to both signals may not be reflected uniformly over time, but may be reflected differently. The first weight (W1) and the second weight (W2) may have different application ratios. Specifically, referring to FIG. 13, the first weight (W1) and the second weight (W2) may have a negative correlation. In addition, the first weight (W1) and the second weight (W2) may have values that vary from 0 (zero) to 1. For example, during days 9, 10, 11, 12, and 13, the first weight (W1) may be 1, 0.9, 0.8, 0.7, and 0.6, respectively, while conversely, the second weight (W2) may be 0, 0.1, 0.2, 0.3, and 0.4. As such, it is not necessary for both weights to change equally.

The above description is based on calculating the corrected biosignal from the corrected continuous biosignal, but the scope of the present disclosure is not limited thereto. As another example, the corrected biosignal may be calculated by applying the first weight (W1) to the 5-minute unit glucose-related biosignal (current value) of the corresponding time point, applying the second weight (W2) to the value of the corresponding time point (the Y-axis value of the rightmost point of the X-axis of the ideal signal as shown in FIG. 12) in the ideal signal calculated by the above-described method, and adding them.

Using the corrected biosignal calculated in this way, the controller 230 may determine input data (step S3), and the controller 230 may derive the biometric data (glucose value) of a specific time point using the input data (step S4).

While the description above is made on the premise that the corresponding time point biosignal is a 5-minute unit glucose-related biosignal with a data collection section of 5 minutes, the scope of the present disclosure is not limited thereto. As another example, the corresponding time point biosignal may be a 1-minute unit glucose-related biosignal with a data collection section of 1 minute. In addition, the corresponding time point biosignal may be a non-glucose-related biosignal.

As described above, according to the embodiment of the present disclosure, in the section after the occurrence of a negative intercept, the blood-related biosignal may be corrected to obtain a corrected biosignal, and the glucose value may be derived using the blood-related biosignal or the corrected biosignal. Accordingly, the glucose value of a specific time point displayed on the terminal in the section after the occurrence of a negative intercept may be displayed more accurately.

## Claims

1. A method of deriving, in an analyte monitoring system (10), biometric data at a specific time point, the method comprising:
acquiring a corresponding time point biosignal, which is a biosignal corresponding to a time point prior to the specific time point (S1);
generating a corrected biosignal from the corresponding time point biosignal when the corresponding time point biosignal satisfies a preset condition (S2), wherein the preset condition is satisfied when the time point of the corresponding time point biosignal has exceeded an end of a preset period of time;
determining one of the corresponding time point biosignal and the corrected biosignal as input data (S3) by:
determining the corrected biosignal as the input data when the corresponding time point biosignal satisfies the preset condition and the corrected biosignal is greater than or equal to the corresponding time point biosignal, and
determining the corresponding time point biosignal as the input data when the corresponding time point biosignal does not satisfy the preset condition or the corrected biosignal is smaller than the corresponding time point biosignal; and
calculating biometric data of the specific time point using the input data and a preset formula (S4).

2. The method according to claim 1, wherein the generating of the corrected biosignal from the corresponding time point biosignal when the preset condition is satisfied (S2) comprises:
acquiring an average value for a plurality of biosignals over a predetermined period of time (S21);
generating an ideal signal by applying the average value to the corresponding time point biosignal (S22);
generating a weight (S23); and
generating the corrected biosignal by applying the weight to the corresponding time point biosignal and the ideal signal (S24, S25).

3. The method according to claim 2, wherein the generating of the ideal signal (S22) comprises:
generating a continuous biosignal with a changing baseline by using the plurality of biosignals including the corresponding time point biosignal (S221);
generating a processed continuous biosignal in which a trend is removed from a waveform of the continuous biosignal (S222); and
applying the average value to the processed continuous biosignal (S223).

4. The method according to claim 3, wherein the generating of the processed continuous biosignal uses a high pass filter (HPF).

5. The method according to claim 3, wherein the continuous biosignal forms a constant baseline in a first period and a descending baseline in a second period.

6. The method according to claim 2, wherein the generating of the weight comprises generating a first weight applied to the corresponding time point biosignal and a second weight applied to the ideal signal,
the first weight and the second weight have a negative correlation, and
the generating of the corrected biosignal comprises generating the corrected biosignal by adding a first weighted signal obtained by applying the first weight to the corresponding time point biosignal and a second weighted signal obtained by applying the second weight to the ideal signal.

7. The method according to claim 6, wherein the first weight and the second weight have values that vary between 0 (zero) and 1.

## Patentansprüche

1. Verfahren zur Ableitung, in einem Analytüberwachungssystem (10), biometrischer Daten zu einem spezifischen Zeitpunkt, wobei das Verfahren Folgendes umfasst:
Erfassen eines entsprechenden Zeitpunktbiosignals, das ein Biosignal entsprechend einem Zeitpunkt vor dem spezifischen Zeitpunkt (S1) ist;
Erzeugen eines korrigierten Biosignals aus dem entsprechenden Zeitpunktbiosignal, wenn das entsprechende Zeitpunktbiosignal eine voreingestellte Bedingung (S2) erfüllt, wobei die voreingestellte Bedingung erfüllt ist, wenn der Zeitpunkt des entsprechenden Zeitpunktbiosignals ein Ende eines voreingestellten Zeitraums überschritten hat;
Bestimmen von einem von dem entsprechenden Zeitpunktbiosignal und dem korrigierten Biosignal als Eingabedaten (S3) durch:
Bestimmen des korrigierten Biosignals als die Eingabedaten, wenn das entsprechende Zeitpunktbiosignal die voreingestellte Bedingung erfüllt und das korrigierte Biosignal größer als das oder gleich dem entsprechenden Zeitpunktbiosignal ist, und
Bestimmen des entsprechenden Zeitpunktbiosignals als die Eingabedaten, wenn das entsprechende Zeitpunktbiosignal die voreingestellte Bedingung nicht erfüllt oder das korrigierte Biosignal kleiner als das entsprechende Zeitpunktbiosignal ist; und
Berechnen biometrischer Daten des spezifischen Zeitpunktes unter Verwendung der Eingabedaten und einer voreingestellten Formel (S4).

2. Verfahren nach Anspruch 1, wobei das Erzeugen des korrigierten Biosignals aus dem entsprechenden Zeitpunktbiosignal, wenn die voreingestellte Bedingung erfüllt ist (S2), Folgendes umfasst:
Erfassen eines Durchschnittswertes für eine Vielzahl von Biosignalen über einen vorbestimmten Zeitraum (S21);
Erzeugen eines idealen Signals durch Anwenden des Durchschnittswertes auf das entsprechende Zeitpunktbiosignal (S22);
Erzeugen eines Gewichts (S23); und
Erzeugen des korrigierten Biosignals durch Anwenden des Gewichts auf das entsprechende Zeitpunktbiosignal und das ideale Signal (S24, S25).

3. Verfahren nach Anspruch 2, wobei das Erzeugen des idealen Signals (S22) Folgendes umfasst:
Erzeugen eines kontinuierlichen Biosignals mit einer sich ändernden Basislinie durch Verwenden der Vielzahl von Biosignalen, beinhaltend das entsprechende Zeitpunktbiosignal (S221);
Erzeugen eines verarbeiteten kontinuierlichen Biosignals, in dem ein Trend von einer Wellenform des kontinuierlichen Biosignals entfernt ist (S222); und
Anwenden des Durchschnittswertes auf das verarbeitete kontinuierliche Biosignal (S223).

4. Verfahren nach Anspruch 3, wobei das Erzeugen des verarbeiteten kontinuierlichen Biosignals einen Hochpassfilter (HPF) verwendet.

5. Verfahren nach Anspruch 3, wobei das kontinuierliche Biosignal eine konstante Basislinie in einer ersten Zeit und eine absteigende Basislinie in einer zweiten Zeit bildet.

6. Verfahren nach Anspruch 2, wobei das Erzeugen des Gewichts Erzeugen eines ersten Gewichts, das auf das entsprechende Zeitpunktbiosignal angewendet wird, und eines zweiten Gewichts, das auf das ideale Signal angewendet wird, umfasst,
das erste Gewicht und das zweite Gewicht eine negative Korrelation aufweisen, und
das Erzeugen des korrigierten Biosignals Erzeugen des korrigierten Biosignals durch Addieren eines ersten gewichteten Signals, das durch Anwenden des ersten Gewichts auf das entsprechende Zeitpunktbiosignal erhalten wird, und eines zweiten gewichteten Signals, das durch Anwenden des zweiten Gewichts auf das ideale Signal erhalten wird, umfasst.

7. Verfahren nach Anspruch 6, wobei das erste Gewicht und das zweite Gewicht Werte aufweisen, die zwischen 0 (Null) und 1 variieren.

## Revendications

1. Procédé de dérivation, dans un système de surveillance d'analyte (10), de données biométriques à un point temporel spécifique, le procédé comprenant :
l'acquisition d'un biosignal de point temporel correspondant, qui est un biosignal correspondant à un point temporel antérieur au point temporel spécifique (S1) ;
la génération d'un biosignal corrigé à partir du biosignal de point temporel correspondant lorsque le biosignal de point temporel correspondant satisfait à une condition prédéfinie (S2), dans lequel la condition prédéfinie est satisfaite lorsque le point temporel du biosignal de point temporel correspondant a dépassé une fin d'une période de temps prédéfinie ;
la détermination de l'un du biosignal de point temporel correspondant et du biosignal corrigé en tant que données d'entrée (S3) par :
la détermination du biosignal corrigé en tant que données d'entrée lorsque le biosignal de point temporel correspondant satisfait à la condition prédéfinie et que le biosignal corrigé est supérieur ou égal au biosignal de point temporel correspondant, et
la détermination du biosignal de point temporel correspondant en tant que données d'entrée lorsque le biosignal de point temporel correspondant ne satisfait pas à la condition prédéfinie ou que le biosignal corrigé est plus petit que le biosignal de point temporel correspondant ; et
le calcul des données biométriques du point temporel spécifique à l'aide des données d'entrée et d'une formule prédéfinie (S4).

2. Procédé selon la revendication 1, dans lequel la génération du biosignal corrigé à partir du biosignal de point temporel correspondant lorsque la condition prédéfinie est satisfaite (S2) comprend :
l'acquisition d'une valeur moyenne pour une pluralité de biosignaux sur une période de temps prédéterminée (S21) ;
la génération d'un signal idéal en appliquant la valeur moyenne au biosignal de point temporel correspondant (S22) ;
la génération d'un poids (S23) ; et
la génération du biosignal corrigé en appliquant le poids au biosignal de point temporel correspondant et au signal idéal (S24, S25).

3. Procédé selon la revendication 2, dans lequel la génération du signal idéal (S22) comprend :
la génération d'un biosignal continu avec une ligne de base variable en utilisant la pluralité de biosignaux, comprenant le biosignal de point temporel correspondant (S221) ;
la génération d'un biosignal continu traité dans lequel une tendance est supprimée d'une forme d'onde du biosignal continu (S222) ; et
l'application de la valeur moyenne au biosignal continu traité (S223).

4. Procédé selon la revendication 3, dans lequel la génération du biosignal continu traité utilise un filtre passe-haut (HPF).

5. Procédé selon la revendication 3, dans lequel le biosignal continu forme une ligne de base constante dans une première période et une ligne de base descendante dans une seconde période.

6. Procédé selon la revendication 2, dans lequel la génération du poids comprend la génération d'un premier poids appliqué au biosignal de point temporel correspondant et d'un deuxième poids appliqué au signal idéal,
le premier poids et le deuxième poids ont une corrélation négative, et
la génération du biosignal corrigé comprend la génération du biosignal corrigé en ajoutant un premier signal pondéré obtenu en appliquant le premier poids au biosignal de point temporel correspondant et un deuxième signal pondéré obtenu en appliquant le deuxième poids au signal idéal.

7. Procédé selon la revendication 6, dans lequel le premier poids et le deuxième poids ont des valeurs qui varient entre 0 (zéro) et 1.
